# EUROPEAN PATENT APPLICATION

(11) **EP 4 681 670 A1**
(43) Date of publication of application: **21.01.2026**
(21) Application number: 25190128.6
(22) Date of filing: 17.07.2025
(51) Int. Cl.: A61B 18/18, A61B 18/00

(54) **MICROWAVE ABLATION PROBE WITH INTEGRATED TEMPERATURE SENSORS**

(30) Priority: 19.07.2024 US 202418778666
(71) Applicant: Varian Medical Systems, Inc., Palo Alto, CA 94304 (US)
(72) Inventor: MAY, Justin, Austin, 78738 (US); GOMEZ, Virgilio, Kyle, 78640 (US)
(74) Representative: Mathisen & Macara LLP

(57) **Abstract**

A microwave ablation probe needle (300) includes a shell (302) extending in an axial direction and defining an inner cavity (306), a tip (304) positioned on a distal end of the shell, and a plurality of thermocouples (308, 310, 312, 314) integrated into the shell.

## Description

### TECHNICAL FIELD

The disclosure relates to microwave ablation probes with integrated temperature sensors.

### BACKGROUND

Microwave ablation probes can be used in clinical treatments such as thermal ablation treatments. In such treatments, thermal ablation can be used to destroy undesirable tissue such as malignant cells in a body. A microwave ablation antenna can be included in the probe and be used to deliver Radio Frequency (RF) energy such as microwave energy to a target tissue to heat the target tissue and destroy the target tissue. The microwave ablation antenna can be positioned inside the ablation probe that can position the microwave ablation antenna proximate the target tissue.

In some treatments, the ablation probe is guided to the target tissue though other tissue or near to tissues or body structures that it is desirable not to damage during treatment. It is desirable, therefore, to maintain a small size of the microwave ablation antenna and/or the needle of the probe. In this manner, damage to tissues and other body structures that may be located close to the target tissue to be destroyed is minimized or prevented. It can be difficult to manufacture ablation probes that are suitably sized for thermal ablation due to the complexity of such devices. Furthermore, it can be difficult to include multiple sensors such as temperature sensors or thermocouples in the ablation probes while also maintaining a suitably small size or suitably small outer diameter. There exists a need, therefore, for improved microwave ablation probes or needle designs that are sufficiently small, deliver known and repeatable ablation zones and can include multiple temperature sensors or thermocouples.

### SUMMARY

The present disclosure is directed to microwave ablation probes that may include multiple temperature sensors or multiple thermocouples. The present invention provides a microwave ablation probe as defined in the appended claims. The microwave ablation probes of the present invention may include needle sizes having an outer diameter in a range of about 1 mm to about 7 mm. The needle may be an elongated member, which may be hollow. The needle may extend in an axial direction to define an internal cavity. The needles of the probes of the present disclosure may have thermocouples that are embedded in the outer wall or integrated into the wall of the needle. In some embodiments, the thermocouples and the electrical leads connected to the thermocouple junction may be printed on the probe needle surface. In other embodiments, the thermocouple junction and the electrical leads may be formed into one or more layers of the probe needle material construction.

In accordance with some embodiments, a microwave ablation probe needle is provided. The microwave ablation probe needle may include a shell or cylindrical wall extending in an axial direction and defining an inner cavity, a tip positioned on a distal end of the shell, and a plurality of thermocouples integrated into the shell. The shell may be formed of fiberglass material, for example. The shell may be formed of multiple different materials joined together. The shell at or near the tip may be formed of a non-metallic or non-conductive material so that, in use, the shell does not interfere with microwaves that are emitted by an antenna positioned in the inner cavity at or near the tip. The tip may be formed of a non-metallic or non-conductive material. The tip may be positioned at a distal end of the shell.

In one aspect, the plurality of thermocouples may be positioned in an array on the shell. The thermocouples may be positioned on an external surface of the needle. The needle may include multiple thermocouples embedded in the shell or integrated into the shell. The term embedded may be used in the present disclosure to describe that the thermocouples are integrally formed with, into, or on the shell. The thermocouples of the present disclosure are not produced separate from the shell and then attached, assembled or connected to the shell. The thermocouples of the present disclosure are not stand alone devices that can be used absent the shell.

In another aspect, each thermocouple of the plurality of thermocouples may be spaced apart from each other. The thermocouples may be used to determine a temperature at or near each thermocouple on the shell.

In another aspect, the plurality of thermocouples may be formed of a metallic ink printed on the shell. In another aspect, the plurality of thermocouples may be formed of a conductive ink printed on the shell.

In another aspect, the plurality of thermocouples may be integrally formed into at least one layer of the shell. In embodiments in which the thermocouples are integrated into the shell, the thermocouples are built into the shell or are produced as part of the shell.

In another aspect, each of the plurality of thermocouples may include a thermocouple junction and a thermocouple lead. The electrical leads may be positioned on the external surface of the needle in some examples. In other examples, the electrical leads may be integrated into the needle. In yet other examples, the electrical leads may be positioned on an internal surface of the needle or in an internal cavity of the needle.

In another aspect, the thermocouple junction and the thermocouple lead may be formed of a metallic ink printed on the shell.

In another aspect, the thermocouple junction may be formed of two different metallic inks.

In another aspect, the thermocouple junction may be formed into an outer layer of the shell.

In another aspect, the thermocouple lead may be formed into an interior layer of the shell.

In another aspect, the shell may comprise multiple layers of fiber glass applied to one another to build up the shell. Each of the layers may include glass fibers that are oriented in different directions relative to each other. In some examples, the shell may be formed using a layer of fiber that are oriented in a longitudinal direction (i.e., in an axial direction, substantially parallel to an axis of the needle) and then applying a layer of fibers to the longitudinal layer that are non-longitudinal (e.g., radially oriented fibers, diagonally-oriented fibers, helically-oriented fibers, etc.). Multiple layers are applied to one another in an alternating fashion to create the shell. In an embodiment, the shell may include a plurality of non-longitudinally wrapped layers of fiberglass and a plurality of longitudinally wrapped layers of fiberglass, and the thermocouple lead may include metallic fibers in at least one of the plurality of longitudinally wrapped layers.

According to another aspect there is provided a microwave ablation probe as defined in the appended claims. In some embodiments, the microwave ablation probe may include a handle connected to the microwave ablation needle with the integrated thermocouples, wherein the thermocouple lead is electrically coupled to the thermocouple and to the handle. The microwave ablation probe may further comprise a supply cable. The handle 204 may connect and/or operably couple aspects of the ablation probe that are provided from the supply cable to the needle. The supply cable may include a power line that delivers the power signal from the microwave generator. The supply cable may also include a coolant supply line and a coolant return line. The coolant supply line may deliver coolant to the needle and the coolant return line may convey the coolant away from the needle. The supply cable may also include one or more wires that may provide signals from one or more temperature sensors, thermocouples, flow sensors, or other sensors that may provide information regarding operating characteristics of the ablation probe. The supply cable may be an elongated tube or conduit and each of the power line, the coolant supply line, the coolant return line, and other wires and lines may extend within an outer shell of the supply cable.

In some embodiments, a microwave ablation probe is provided. The microwave ablation probe may include a handle connected to the microwave ablation probe needle with integrated thermocouples, wherein the thermocouple lead extends from the thermocouple to the handle.

In another there is provided a microwave ablation apparatus as defined in the appended claims. The microwave ablation apparatus may include an ablation console comprising a microwave generator, and an ablation probe coupled to the ablation console. The ablation probe may include a probe needle that includes a shell extending in an axial direction and defining an inner cavity, a tip positioned at a distal end of the shell, and a plurality of thermocouples integrated into the shell. The microwave generator that may provide a power signal that is sent to an antenna in the needle of the ablation probe. The power signal may be provided to the antenna via a supply cable that may extend from the ablation console to the ablation probe. The supply cable may also provide a coolant to the ablation probe. The coolant may be a saline solution or other coolant that may be supplied to the ablation probe from a coolant receptacle via a pump cartridge or other pump mechanism.

In another aspect, a method of making a microwave ablation probe needle is provided, as defined in the appended claims. The method may include forming a shell, the shell extending in an axial direction and having an outer surface, and depositing metallic ink on the outer surface of the shell to form a thermocouple.

In one aspect, the step of depositing metallic ink may include printing the thermocouple using a printer head.

In another aspect, the step of depositing the ink may include depositing two different metallic inks to form a thermocouple junction and a thermocouple lead.

In another aspect, the thermocouple lead may include a continuous electrical path from the thermocouple to a handle of a microwave ablation probe.

In some embodiments a method of making a microwave ablation probe needle is provided. The method may include forming a plurality of longitudinal layers of fiberglass and a plurality of non-longitudinal layers of fiberglass to define a shell extending in a longitudinal direction, and embedding a thermocouple into the shell by applying metallic fibers into at least one of the plurality of longitudinal layers and into at least one of the plurality of non-longitudinal layers.

In another aspect, the thermocouple may include a thermocouple lead extending from a thermocouple junction to a handle of a microwave ablation probe.

In another aspect, the thermocouple lead may be formed by applying the metallic fibers in the at least one of the plurality of longitudinal layers.

Further areas of applicability will become apparent from the description provided herein. The description and specific examples in this summary are intended for purposes of illustration only and are not intended to limit the scope of the present disclosure.

### BRIEF DESCRIPTION OF THE DRAWINGS

The features and advantages of the present disclosures will be more fully disclosed in, or rendered apparent by the following detailed descriptions of example embodiments. The detailed descriptions of the example embodiments are to be considered together with the accompanying drawings wherein like numbers refer to like parts and further wherein:
FIG. 1 is an isometric view of an example microwave ablation apparatus in accordance with some embodiments of the present disclosure.
FIG. 2 is an example microwave ablation probe in accordance with some embodiments of the present disclosure.
FIG. 3 is an illustration of an example portion of a needle that includes multiple thermocouples printed on the needle in accordance with some embodiments of the present disclosure.
FIG. 4 is an illustration of an example portion of a needle that includes multiple thermocouples embedded in layers of the needle in accordance with some embodiments of the present disclosure.

### DETAILED DESCRIPTION

The description of the preferred embodiments is intended to be read in connection with the accompanying drawings, which are to be considered part of the entire written description of these disclosures. While the present disclosure is susceptible to various modifications and alternative forms, specific embodiments are shown by way of example in the drawings and will be described in detail herein. The objectives and advantages of the claimed subject matter will become more apparent from the following detailed description of these exemplary embodiments in connection with the accompanying drawings.

It should be understood, however, that the present disclosure is not intended to be limited to the particular forms disclosed. Rather, the present disclosure covers all modifications, equivalents, and alternatives that fall within the spirit and scope of these exemplary embodiments. The terms "couple," "coupled," "operatively coupled," "operatively connected," and the like should be broadly understood to refer to connecting devices or components together either mechanically, electrically, wired, wirelessly, or otherwise, such that the connection allows the pertinent devices or components to operate (e.g., communicate) with each other as intended by virtue of that relationship.

In the present disclosure the singular forms "a," "an," and "the" include the plural reference, and reference to a particular numerical value includes at least that particular value, unless the context clearly indicates otherwise. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", "2-5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included." It is intended that any component, element, attribute, or step that is positively recited herein may be explicitly excluded in the claims, whether such components, elements, attributes, or steps are listed as alternatives or whether they are recited in isolation.

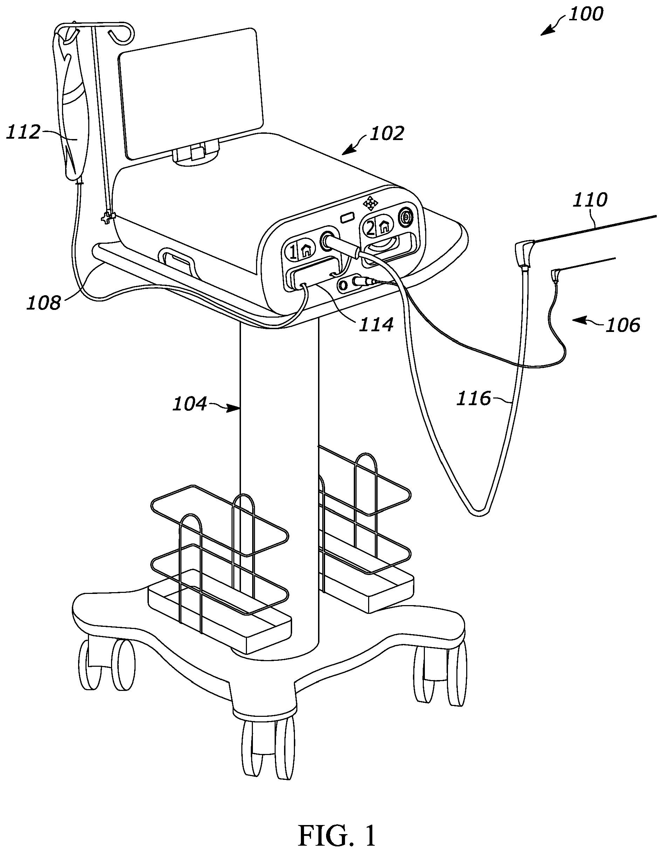

Turning now to FIG. 1, an example microwave ablation apparatus 100 is shown. In this example, the ablation apparatus 100 includes an ablation console 102, and an ablation probe 110. The ablation console 102 may be portable, in some examples, and may be positioned on a platform 108 of stand 104 to allow the console 102 to be easily moved and positioned as desired during treatment. The console 102 may include a microwave generator that may provide a power signal that is sent to an antenna in the needle of the ablation probe 110. The power signal may be provided to the antenna via a supply cable 116 that may extend from the console 102 to the ablation probe 110. The supply cable 116 may also provide a coolant to the ablation probe 110. The coolant may be a saline solution or other coolant that may be supplied to the ablation probe 110 from a coolant receptacle 112 via a pump cartridge 114 or other pump mechanism.

During an ablation treatment, the needle of the ablation probe 110 may be positioned at or near a target tissue in a patient. A power signal may be supplied to the ablation probe 110 from the console 102. The power signal may be supplied to the ablation probe 110 via the supply cable 116. The power signal may cause microwaves to be emitted from an antenna in the needle of the ablation probe 110. The microwaves may cause the tissue at the needle to be heated to a suitable temperature to destroy the target tissue. A temperature sensor 106 may be positioned at or near the target tissue to measure a temperature that may be achieved at or near the ablation zone. In addition, and as further described below, the needle or the handle of the ablation probe 110 may include other thermocouples or temperature probes to provide information regarding temperatures of the coolant or the needle.

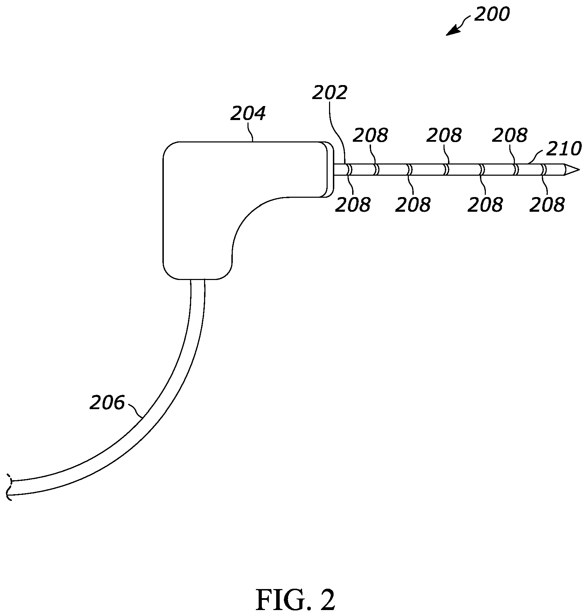

Referring now to FIG. 2, an example ablation probe 200 is shown. The ablation probe 200 may be used with the microwave ablation apparatus 100 previously described. The ablation probe 200 may include a needle 202, a handle 204, and a supply cable 206. The ablation probe 200 may be a disposable or single-use probe that is used during a single procedure or for a single patient. In other examples, the ablation probe 200 (or portions of the ablation probe 200) may be re-used.

The needle 202 of the ablation probe 200 may be an elongated member that maybe inserted into a patient at or near the target tissue. The needle 202 may be a metal, fiberglass, composite, alloy or other rigid material. The needle 202 may be cylindrical and pointed. The needle 202 may be hollow such that various elements are positioned inside the needle 202. For example, a power line and an antenna may be located inside needle 202. The power line may be positioned inside the needle 202 to deliver a power signal from a microwave generator to the antenna. Coolant lines may also be positioned inside the needle to deliver coolant to a distal end of the needle 202. The coolant may be used to control a temperature of the needle 202 and/or to control a size and shape of the ablation zone that may be created at the distal end of the needle 202.

The handle 204 may connect and/or operably couple aspects of the ablation probe 200 that are provided from the supply cable 206 to the needle 202. The supply cable 206 may include a power line that delivers the power signal from the microwave generator. The supply cable 206 may also include a coolant supply line and a coolant return line. The coolant supply line may deliver coolant to the needle 202 and the coolant return line may convey the coolant away from the needle 202 after it has been used to cool the needle 202. The supply cable 206 may also include one or more wires that may be provide signals from one or more temperature sensors, thermocouples, flow sensors, or other sensors that may provide information regarding operating characteristics of the ablation probe 200.

The supply cable 206 may be an elongated tube or conduit and each of the power line, the coolant supply line, the coolant return line, and other wires and lines may extend within an outer shell of the supply cable 206. A length of the supply cable 206 may allow a user of the ablation probe 200 to position the needle 202 of the ablation probe 200 as desired and to allow the patient to be moved in and/or out of an imaging device during a treatment. For example, a location of the needle 202 may be determined using CT, MRI, Ultrasound, or other imaging devices before, during, or after treatment is performed. Thus, the supply cable 206 may be sufficiently long to allow these diagnostic procedures to be performed. In some examples, the supply cable 206 is at least 12 feet in length. In other examples, the supply cable 206 is at least 10 feet in length. In other examples, other lengths may be used.

The handle 204 may connect and/or operably couple the supply cable 206 to the needle 202. The power signal, the coolant, and/or sensor signals may be conveyed or delivered from the supply cable 206 to the needle 202 through the handle 204. The handle 204 may include a housing that may enclose various aspects such as fluid connections, electrical connections, electrical components, and the like. The handle 204 may be configured to orient the needle 202 in a desired alignment relative to the longitudinal axis of the supply cable 206. In the example shown, the handle 204 is a right-angle handle. The handle 204 orients the needle 202 at about 90 degrees (or substantially perpendicular) to the longitudinal axis of the supply cable 206. In other examples, the handle 204 may orient the needle 202 at a different angle relative to the longitudinal axis of the supply cable 206.

The needle 202 may include multiple temperature sensors positioned at different positions. The needle, in this example, includes multiple thermocouples 208 positioned at different axial positions along an external surface of the needle 202. The microwave ablation probe 200 may also include other temperature sensors or other thermocouples located at other locations in the handle 204, and/or inside the needle 202. The thermocouples 208 may be positioned on an external surface 210 of the needle 202 to measure a temperature at different axial locations. The temperature measurements may be used to determine an ablation zone that is created when the microwaves are emitted during operation of the probe 200. The thermocouples 208 may measure the temperature at an axial location or may be configured at measurement points that can locally measure the temperature at a particular point along the axial surface 210 and at a particular circumferential location.

The thermocouples 208 may be electrically coupled to the handle 204 and/or to the supply cable 206 via the handle 204. The thermocouples 208 may provide measurement data regarding a temperature of the needle 202 to the ablation console 102. The console 102 may, in turn, utilize the measurement data and/or display the measurement data to the user. The thermocouples 208 may be electrically coupled to the other aspects of the ablation probe 200 and/or the ablation apparatus 100 via an electrical lead or electrical wire. The measurement data may be provided via the electrical leads. The electrical leads may be positioned on the external surface 210 of the needle 202 in some examples. In other examples, the electrical leads may be integrated into the needle 202. In yet other examples, the electrical leads may be positioned on an internal surface of the needle 202 or in an internal cavity of the needle 202.

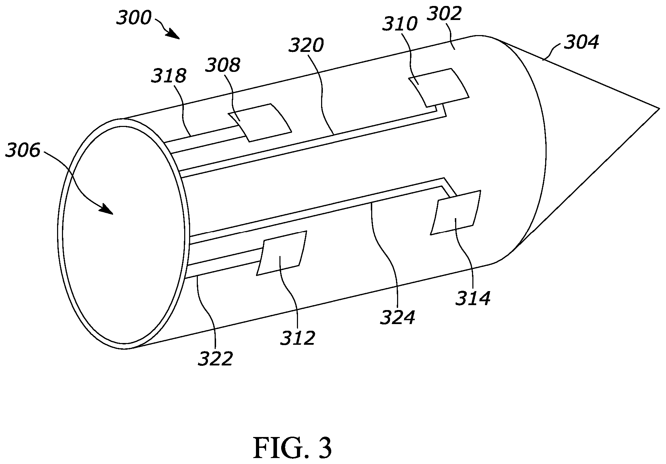

Turning now to FIG. 3, an example needle 300 is shown. The needle 300 may be used in the microwave ablation probe 200 previously described. A portion of the needle 300 is illustrated but it should be appreciated that the needle 300 may extend from a handle of the microwave ablation probe. Furthermore, the needle 300 is illustrated without other internal elements that may be included in a microwave ablation probe such as a power line with antenna and a coolant conduit.

Needle 300 may include a shell 302 and a tip 304. The shell 302 may be formed of a suitable material and may extend in an axial direction to define an internal cavity 306. The shell 302 may be formed of a fiberglass material for example. In other examples, the shell 302 may be formed of multiple different materials joined together. The shell 302 at or near the tip 304 is formed of a non-metallic or non-conductive material so that the shell 302 does not interfere with the microwaves that are emitted by an antenna positioned in the inner cavity 306 at or near the tip 304.

The tip 304 may be positioned at a distal end of the shell 302. The tip 304 may be a leading end of the needle 300 as the needle 300 is inserted into a patient at or near a target tissue. The tip may also be formed of a non-metallic or non-conductive material. The tip 304 may be formed of a non-metallic or non-conductive material so as to not interfere with the microwaves that are produced at the antenna positioned in the internal cavity 306 of the shell 302. In some examples, the tip 304 may be formed of a ceramic material. In other examples, other materials may be used.

The needle 300 may also include multiple thermocouples embedded in the shell 302 or integrated into the shell 302. The term embedded may be used in the present disclosure to describe that the thermocouples are integrally formed with, into, or on the shell 302. In embodiments in which the thermocouples are integrated into the shell 302, the thermocouples are built into the shell or are produced as part of the shell 302. The thermocouples of the present disclosure are not produced separate from the shell 302 and then attached, assembled or connected to the shell 302. The thermocouples of the present disclosure are not stand alone devices that can be used absent the shell 302. The thermocouples of the present disclosure may be embedded into or integrated on the shell 302 using various processes or techniques that will be further described below. In various embodiments of the present disclosure, the thermocouples may be embedded by printing the thermocouples on the shell 302 using a metallic or conductive ink. In other embodiments of the present disclosure, the thermocouples may be embedded by forming the thermocouples into one or more layers of material that applied to each other, such as during the forming of a fiberglass shell 302.

In the example shown, the shell 302 may include a first thermocouple 308, a second thermocouple 310, a third thermocouple 312, and a fourth thermocouple 314. Each of the thermocouples may be positioned on a different location on the shell 302. The thermocouples may be used to determine a temperature at or near each thermocouple on the shell 302. Each thermocouple may include a thermocouple junction and a thermocouple lead. The thermocouple junction may comprise two different metallic or conductive materials joined together. The voltage produced by the junction of two dissimilar conductive materials may be used to determine a temperature at the location of the thermocouple junction. The thermocouple leads may extend away from the thermocouple junction and may be used to measure the voltage at a location away from the thermocouple junction. The two dissimilar metals may be any suitable pair of dissimilar conductive materials such as chromel-constantan, iron-constantan, chromel-alumel, nicrosil-nisil, copper-constantan, or other pairs of conductive materials known to one skilled in the art.

The thermocouple leads may extend from each of the first thermocouple 308, the second thermocouple 310, the third thermocouple 312, and the fourth thermocouple 314. In this example, a first thermocouple lead 318 may be electrically coupled to the first thermocouple 308 and may extend from the first thermocouple 308 to an electrical wire, electrical bus, or other electrical component in the handle 204 of the microwave ablation probe 200. A second thermocouple lead 320 may be electrically coupled to the second thermocouple 310 and may extend from the second thermocouple 310 to an electrical wire, electrical bus, or other electrical component in the handle 204 of the microwave ablation probe 200. A third thermocouple lead 322 may be electrically coupled to the third thermocouple 312 and may extend from the third thermocouple 312 to an electrical wire, electrical bus, or other electrical component in the handle 204 of the microwave ablation probe 200. A fourth thermocouple lead 324 may be electrically coupled to the fourth thermocouple 314 and may extend from the fourth thermocouple 314 to an electrical wire, electrical bus, or other electrical component in the handle 204 of the microwave ablation probe 200. While four thermocouples are shown in this example, it should be appreciated that the needle 300 may include more or less than four thermocouples and may be arranged in a different pattern or array along the shell 302.

The thermocouples and the thermocouple leads may be printed onto a surface of the shell 302 using a metallic or conductive ink. The metallic ink may comprise the dissimilar conductive materials previously described. The metallic ink may be deposited by a suitable printer head. The metallic ink, in one example, may be deposited in a liquid form and allowed to harden or cure. In other examples, the metallic ink may be deposited using a piezoelectric printer head.

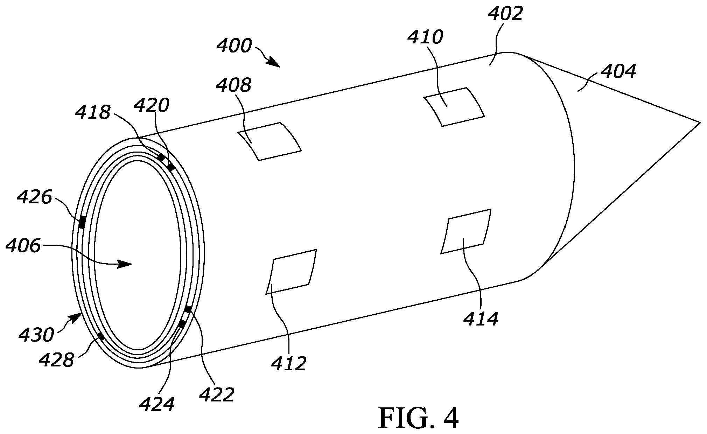

Referring now to FIG. 4, another example needle 400 is shown. For illustration purposes, a portion of the needle 400 is shown. The needle 400 may be similar to the needle 300 previously described in that the needle 400 includes a shell 402 and a tip 404. The shell 402 may define an internal cavity 406 in which other elements of the microwave ablation probe can be positioned, such as a power line with microwave antenna and a coolant conduit (not shown). In this example, the shell 402 may include thermocouples embedded into the shell 402 using a different process from the printing process previously described.

In the example shown, the shell 402 may include a first thermocouple 408, a second thermocouple 410, a third thermocouple 412, and a fourth thermocouple 414. The thermocouples may include a thermocouple junction of dissimilar conductive materials as previously described and thermocouple leads electrically coupled to the thermocouples that are used to measure of voltage at a position away from the thermocouple to determine temperature. In this example, the thermocouple junctions and the thermocouple leads may be integrally formed into the shell 402 during the fabrication of the shell 402.

In this example, the shell 402 may be formed by applying a plurality of layers 430 of fiberglass to one another. The multiple layers of fiber glass may be applied to one another to build up the shell 402. Each of the layers may include glass fibers that are oriented in different directions relative to each other. By orienting the fibers in different directions, the strength of the overall shell 402 is increased as compared to a structure in which all the fibers are oriented in the same direction. In some examples, the shell 402 is formed using a layer of fiber that are oriented in a longitudinal direction (i.e., in an axial direction, substantially parallel to an axis of the needle 402) and then applying a layer of fibers to the longitudinal layer that are non-longitudinal (e.g., radially oriented fibers, diagonally-oriented fibers, helically-oriented fibers, etc.). Multiple layer 430 are applied to one another in an alternating fashion to create the shell 402.

During such a process of fabricating the shell 402, metallic or conductive fibers or materials may be deposited in one or more of the layers 430 to form the thermocouples and/or the thermocouple leads. Shown at the end of the needle 402 are thermocouple leads 418, 420, 422, 424, 426, and 428. The thermocouple leads 418, 420, 422, 424, 426, and 428 may be formed by using a fiber or wire of metallic or conductive material and applying such fiber or wire during the formation of a longitudinal layer of fiberglass during formation of shell 402. In this manner, the leads 418, 420, 422, 424, 426, and 428 may extend along the longitudinal length of the shell 402 and may electrically couple one of the thermocouples 408, 410, 412, 414 to the handle 204 of the microwave ablation probe 200. The thermocouple leads 418, 420, 422, 424, 426, and 428 may be positioned and/or formed from an internal layer (i.e., not exposed to the exterior of the shell 402) and the thermocouple junctions may be formed in an external layer of the shell 402.

The following is a list of non-limiting illustrative embodiments disclosed herein:
Illustrative embodiment 1: A microwave ablation probe needle comprising: a shell extending in an axial direction and defining an inner cavity; a tip positioned on a distal end of the shell; and a plurality of thermocouples integrated into the shell.
Illustrative embodiment 2: The microwave ablation probe needle of illustrative embodiment 1, wherein the plurality of thermocouples are positioned in an array on the shell.
Illustrative embodiment 3: The microwave ablation probe needle of any of illustrative embodiments 1 or 2, wherein each thermocouple of the plurality of thermocouples is spaced apart from each other.
Illustrative embodiment 4. The microwave ablation probe needle of any of illustrative embodiments 1 to 3, wherein the plurality of thermocouples comprise a metallic ink printed on the shell.
Illustrative embodiment 5. The microwave ablation probe needle of any of illustrative embodiments 1 to 3, wherein the plurality of thermocouples are integrally formed into at least one layer of the shell.
Illustrative embodiment 6. The microwave ablation probe needle of any of illustrative embodiments 1 to 5, wherein each of the plurality of thermocouples comprise a thermocouple junction and a thermocouple lead.
Illustrative embodiment 7. The microwave ablation probe needle of illustrative embodiment 6, wherein the thermocouple junction and the thermocouple lead comprise a metallic ink printed on the shell.
Illustrative embodiment 8. The microwave ablation probe needle of any of illustrative embodiments 6 or 7, wherein the thermocouple junction comprises two different metallic inks.
Illustrative embodiment 9. The microwave ablation probe needle of illustrative embodiment 6, wherein the thermocouple junction is formed into an outer layer of the shell.
Illustrative embodiment 10. The microwave ablation probe needle of any of illustrative embodiments 6 or 9, wherein the thermocouple lead is formed into an interior layer of the shell.
Illustrative embodiment 11. The microwave ablation probe needle of any of illustrative embodiments 6, 9, or 10, wherein the shell comprises a plurality of non-longitudinally wrapped layers of fiberglass and a plurality of longitudinally wrapped layers of fiberglass, and the thermocouple lead comprises metallic fibers in at least one of the plurality of longitudinally wrapped layers.
Illustrative embodiment 12. A microwave ablation probe comprising a handle connected to the microwave ablation needle of illustrative embodiment 6, wherein the thermocouple lead is electrically coupled to the thermocouple and to the handle.
Illustrative embodiment 13. A microwave ablation apparatus comprising: an ablation console comprising a microwave generator; and an ablation probe coupled to the ablation console, the ablation probe including a probe needle comprising: a shell extending in an axial direction and defining an inner cavity; a tip positioned at a distal end of the shell; and a plurality of thermocouples integrated into the shell.
Illustrative embodiment 14. A method of making a microwave ablation probe needle comprising: forming a shell, the shell extending in an axial direction and having an outer surface; and depositing metallic ink on the outer surface of the shell to form a thermocouple.
Illustrative embodiment 15. The method of illustrative embodiment 14, wherein the step of depositing metallic ink comprises printing the thermocouple using a printer head.
Illustrative embodiment 16. The method of illustrative embodiment 15, wherein the step of depositing the ink comprises depositing two different metallic inks to form a thermocouple junction and a thermocouple lead.
Illustrative embodiment 17. The method of illustrative embodiment 16, wherein the thermocouple lead comprises a continuous electrical path from the thermocouple to a handle of a microwave ablation probe.
Illustrative embodiment 18. A method of making a microwave ablation probe needle comprising: forming a plurality of longitudinal layers of fiberglass and a plurality of non-longitudinal layers of fiberglass to define a shell extending in a longitudinal direction; and embedding a thermocouple into the shell by applying metallic fibers into at least one of the plurality of longitudinal layers and into at least one of the plurality of non-longitudinal layers.
Illustrative embodiment 19. The method of illustrative embodiment 18, wherein the thermocouple comprises a thermocouple lead extending from a thermocouple junction to a handle of a microwave ablation probe.
Illustrative embodiment 20. The method of illustrative embodiment 19, wherein the thermocouple lead is formed by applying the metallic fibers in the at least one of the plurality of longitudinal layers.

The foregoing is provided for purposes of illustrating, explaining, and describing embodiments of these disclosures. Modifications and adaptations to these embodiments will be apparent to those skilled in the art and may be made without departing from the scope or spirit of these disclosures.

## Claims

1. A microwave ablation probe needle (202, 300, 400) comprising:
a shell (302, 402) extending in an axial direction and defining an inner cavity (306, 406);
a tip (304, 404) positioned on a distal end of the shell; and
a plurality of thermocouples (208, 308, 310, 312, 314, 408, 410, 412, 414) integrated into the shell.

2. The microwave ablation probe needle of claim 1, wherein the plurality of thermocouples (208, 308, 310, 312, 314, 408, 410, 412, 414) are positioned in an array on the shell (302) and, optionally, each thermocouple of the plurality of thermocouples is spaced apart from each other.

3. The microwave ablation probe needle of claim 1 or 2, wherein the plurality of thermocouples comprise a metallic ink printed on the shell.

4. The microwave ablation probe needle of any one of claims 1 to 3, wherein the plurality of thermocouples are integrally formed into at least one layer of the shell.

5. The microwave ablation probe needle according to any one of the preceding claims, wherein each of the plurality of thermocouples (208, 308, 310, 312, 314, 408, 410, 412, 414) comprise a thermocouple junction and a thermocouple lead (318, 320, 322, 324, 418, 420, 422, 424, 426, 428).

6. The microwave ablation probe needle of claim 5, wherein the thermocouple junction and the thermocouple lead (318, 320, 322, 324) comprise a metallic ink printed on the shell and, optionally, the thermocouple junction comprises two different metallic inks.

7. The microwave ablation probe needle of claim 5 or 6, wherein the thermocouple junction is formed into an outer layer of the shell.

8. The microwave ablation probe needle of claim 5 or 7, wherein the thermocouple lead (318, 320, 322, 324) is formed into an interior layer of the shell.

9. The microwave ablation probe needle of claim 5,7 or 8, wherein the shell (402) comprises a plurality of non-longitudinally wrapped layers of fiberglass and a plurality of longitudinally wrapped layers of fiberglass, and the thermocouple lead (418, 420, 422, 424, 426, 428) comprises metallic fibers in at least one of the plurality of longitudinally wrapped layers (430).

10. A microwave ablation probe comprising a handle connected to the microwave ablation needle of any one of the preceding claims, wherein the thermocouple lead (318, 320, 322, 324) electrically couples the thermocouple to the handle (204).

11. A microwave ablation apparatus (100) comprising:
an ablation console (102) comprising a microwave generator; and
an ablation probe (110, 200) coupled to the ablation console, the ablation probe including a probe needle (202, 300) comprising:
a shell (302, 402) extending in an axial direction and defining an inner cavity (306, 406);
a tip (304, 4040 positioned at a distal end of the shell; and
a plurality of thermocouples (208, 308, 310, 312, 314, 408, 410, 412, 414) integrated into the shell.

12. A method of making a microwave ablation probe needle (202, 300) comprising:
forming a shell, the shell extending in an axial direction and having an outer surface (210); and
integrating a thermocouple (208, 308, 310, 312, 314, 408, 410, 412, 414) on a surface (210) of the shell (302, 402) or into the shell to form a thermocouple.

13. The method of claim 12, wherein the step of integrating the thermocouple comprises printing the thermocouple in a metallic ink using a printer head and , optionally, the step of printing the thermocouple (208, 308, 310, 312, 314) comprises depositing two different metallic inks to form a thermocouple junction and a thermocouple lead (318, 320, 322, 324), and, optionally, the thermocouple lead (318, 320, 322, 324) comprises a continuous electrical path from the thermocouple (208, 308, 310, 312, 314) to a handle (204) of a microwave ablation probe (200).

14. The method of claim 12, wherein the step of integrating the thermocouple (408, 410, 412, 414) comprises embedding the thermocouple (408, 410, 412, 414) into the shell by applying metallic fibers into at least one of a plurality of longitudinal layers (430) and into at least one of a plurality of non-longitudinal layers (430).

15. The method of claim 14, wherein the thermocouple (408, 410, 412, 414) comprises a thermocouple lead (418, 420, 422, 424, 426, 428) extending from a thermocouple junction to a handle (204) of a microwave ablation probe (200 and, optionally, the thermocouple lead (418, 420, 422, 424, 426, 428) is formed by applying metallic fibers in the at least one of the plurality of longitudinal layers (430).
